# EUROPEAN PATENT APPLICATION

(11) **EP 4 134 446 A1**
(43) Date of publication of application: **15.02.2023**
(21) Application number: 21194884.9
(22) Date of filing: 03.09.2021
(51) Int. Cl.: C12Q 1/6876

(54) **METHOD FOR GENETIC SELECTION BASED ON INCREASED REPRODUCTIVE TRAITS**

(30) Priority: 13.08.2021 PT 2021117401
(71) Applicant: Cebal - Centro De Biotecnologia Agrícola E Agro-Alimentar Do Alentejo, 7800-309 Beja (PT)
(72) Inventor: COSTA DO AMARAL RAMOS, ANTÓNIO MARCOS, 7800-309 BEJA (PT); USIÉ CHIMENOS, ANA ISABEL, 7800-309 BEJA (PT); BRANCO GASPAR, DANIEL FILIPE, 7800-309 BEJA (PT); PINHEIRO RIBEIRO DE MEIRELES, BRÍGIDA DA NATIVIDADE, 2685-019 SACAVÉM (PT); CARVALHO MAGALHÃES, HUGO, 4700-284 BRAGA (PT); SANTOS BARBOSA, PEDRO, 1000-127 LISBOA (PT); FIALHO LEÃO, CÉLIA CRISTINA, 2780-157 OEIRAS (PT)
(74) Representative: Neves, Ana

(57) **Abstract**

The present patent application discloses a method for the genetic selection of animals from the Alentejano pig breed with a profile associated with increased reproductive traits. Said profile is determined based on a group of Single Nucleotide Polymorphisms (SNPs) herein associated with increased reproductive traits in the Alentejano pig breed.

## Description

### Technical field

The present patent application relates to a method for the genetic selection of animals from the Alentejano pig breed with a profile associated with increased reproductive traits.

### Background art

The Alentejano pig breed is characterized by low prolificacy, high number of non-productive days and high mortality rate of pre-weaned piglets. When combined with the long production cycle characteristic of the breed, these factors are responsible for reduced productivity values per sow, evaluated as the number of weaned piglets per sow per year. This evaluation turns out to be a simple way to analyze a very complex quantitative characteristic, in which several physiological factors contribute to the phenotypic variation observed. Still, from the genetic improvement point of view this is an attractive trait, since it is easily measurable and can be collected repeatedly on the same animal. Moreover, the use of molecular markers is especially important in this case, since productivity during the productive life of a female, which is also closely linked to longevity, can only be fully evaluated after the productive cycle is over. To the present date no genetic markers have been described in genes associated with reproductive traits for the Alentejano Pig breed. Therefore, the use of this type of technology provides a greater component of innovation, and also offers the possibility of increasing the genetic progress observed in the breed for this type of trait.

### Summary

The present patent application relates to a method for the genetic selection of animals from the Alentejano pig breed with a profile associated with increased reproductive traits.

The method for selection of animals from the Alentejano pig breed based on genotypes with increased reproductive traits comprises the following steps:
Preparation of an Alentejano pig biological sample and extraction of DNA from said sample;
Genotyping the DNA sample for at least one of the following Single Nucleotide Polymorphisms (SNPs),

| Chromosome | Position | Gene symbol | Reference allele | Alternative allele |
|---|---|---|---|---|
| 3 | 121,822,828 | DDX1 | A | G |
| 3 | 121,833,982 | DDX1 | T | C |
| 3 | 121,853,941 | DDX1 | C | A |
| 3 | 121,853,948 | DDX1 | G | A |
| 5 | 38,530,545 | KCNC2 | G | C |
| 6 | 146,829,589 | LEPR | G | A |
| 6 | 146,830,356 | LEPR | C | T |
| 6 | 146,831,587 | LEPR | C | T |
| 6 | 146,861,105 | LEPR | A | G |
| 12 | 15,407,672 | ACE | A | G |
| 12 | 15,407,684 | ACE | T | C |
| 14 | 97,104,037 | MBL2 | A | G |

Determining the pig's reproductive profile based on the presence of at least one of said SNPs significantly associated with increased reproductive traits.

In one embodiment, the sample analyzed is preferably a blood sample.

In another embodiment, the above-mentioned Single Nucleotide Polymorphyms (SNPs) are used as biomarkers of increased reproductive traits in Alentejano pig breed.

### Detailed Description

The present patent application discloses a method for the genetic selection of animals from the Alentejano pig breed with profile associated with increased reproductive traits. Said profile is determined based on a group of Single Nucleotide Polymorphisms (SNPs) disclosed herein which are associated with increased reproductive traits in the Alentejano pig breed.

The technology disclosed herein is based on the sequencing of genomes of animals of the Porco Alentejano breed, to identify the global set of variations, namely SNPs, present in the genome of each animal, in particular the variation present in genes associated with reproductive traits. Twelve SNPs located in 5 different genes are identified herein, namely in the DDX1 (DEAD-Box Helicase 1), KCNC2 (Potassium Voltage-Gated Channel Subfamily C Member 2), LEPR (Leptin receptor), ACE (Angiotensin I Converting Enzyme) and MBL2 (Mannose Binding Lectin 2) genes. The complete list of these SNPs is shown in Table 1.

The 12 SNPs identified are described in Table 1.

| Chromosome | Position | Gene ID | Gene symbol | Reference allele | Alternative allele |
|---|---|---|---|---|---|
| 3 | 121,822,828 | ENSSSCG00000008619 | DDX1 | A | G |
| 3 | 121,833,982 | ENSSSCG00000008619 | DDX1 | T | C |
| 3 | 121,853,941 | ENSSSCG00000008619 | DDX1 | C | A |
| 3 | 121,853,948 | ENSSSCG00000008619 | DDX1 | G | A |
| 5 | 38,530,545 | ENSSSCG00000000516 | KCNC2 | G | C |
| 6 | 146,829,589 | ENSSSCG00000025188 | LEPR | G | A |
| 6 | 146,830,356 | ENSSSCG00000025188 | LEPR | C | T |
| 6 | 146,831,587 | ENSSSCG00000025188 | LEPR | C | T |
| 6 | 146,861,105 | ENSSSCG00000025188 | LEPR | A | G |
| 12 | 15,407,672 | ENSSSCG00000017296 | ACE | A | G |
| 12 | 15,407,684 | ENSSSCG00000017296 | ACE | T | C |
| 14 | 97,104,037 | ENSSSCG00000010427 | MBL2 | A | G |

The method of the present patent application involves several steps. Initially, it is necessary to collect a sample of biological material, such as a blood sample, for DNA extraction. Next, this DNA sample is genotyped for the SNPs of interest, and the genotypes obtained for each SNP are evaluated. The decision to select the animal is based on the presence of the genotype (s) significantly associated with increased reproductive traits.

The method for selection of animals from the Alentejano pig breed based on genotypes with increased reproductive traits comprises the following steps:
Preparation of an Alentejano pig biological sample and extraction of DNA from said sample;
Genotyping the DNA sample for at least one of the following Single Nucleotide Polymorphisms (SNPs),

| Chromosome | Position | Gene symbol | Reference allele | Alternative allele |
|---|---|---|---|---|
| 3 | 121,822,828 | DDX1 | A | G |
| 3 | 121,833,982 | DDX1 | T | C |
| 3 | 121,853,941 | DDX1 | C | A |
| 3 | 121,853,948 | DDX1 | G | A |
| 5 | 38,530,545 | KCNC2 | G | C |
| 6 | 146,829,589 | LEPR | G | A |
| 6 | 146,830,356 | LEPR | C | T |
| 6 | 146,831,587 | LEPR | C | T |
| 6 | 146,861,105 | LEPR | A | G |
| 12 | 15,407,672 | ACE | A | G |
| 12 | 15,407,684 | ACE | T | C |
| 14 | 97,104,037 | MBL2 | A | G |

Determining the pig's reproductive profile based on the presence of at least one of said SNPs significantly associated increased reproductive traits.

In one embodiment, the sample analyzed is preferably a blood sample.

## Claims

1. Method for selection of animals from the Alentejano pig breed based on genotypes with increased reproductive traits comprising the following steps:
Preparation of an Alentejano pig biological sample and extraction of DNA from said sample;
Genotyping the DNA sample for at least one of the following Single Nucleotide Polymorphisms (SNPs),
| Chromosome | Position | Gene symbol | Reference allele | Alternative allele |
|---|---|---|---|---|
| 3 | 121,822,828 | DDX1 | A | G |
| 3 | 121,833,982 | DDX1 | T | C |
| 3 | 121,853,941 | DDX1 | C | A |
| 3 | 121,853,948 | DDX1 | G | A |
| 5 | 38,530,545 | KCNC2 | G | C |
| 6 | 146,829,589 | LEPR | G | A |
| 6 | 146,830,356 | LEPR | C | T |
| 6 | 146,831,587 | LEPR | C | T |
| 6 | 146,861,105 | LEPR | A | G |
| 12 | 15,407,672 | ACE | A | G |
| 12 | 15,407,684 | ACE | T | C |
| 14 | 97,104,037 | MBL2 | A | G |
Determining the pig's reproductive profile based on the presence of at least one of said SNPs significantly associated with increased reproductive traits.

2. Single Nucleotide Polymorphyms (SNPs) as defined in claim 1 for use as biomarkers of increased reproductive traits in Alentejano pig breed animals.
